# EUROPEAN PATENT APPLICATION

(11) **EP 1 504 792 A1**
(43) Date of publication of application: **09.02.2005**
(21) Application number: 03384003.4
(22) Date of filing: 08.08.2003
(51) Int. Cl.: A61N 5/06

(54) **Tanning system**

(71) Applicant: Enco Electronics, S.A., 08221 Terrassa (ES)
(72) Inventor: Boncompte, Joan Francesc Casas, 08221 Terrassa (ES)
(74) Representative: Marques Alos, Fernando

(57) **Abstract**

This invention consists of a progressive tanning system comprising an artificial tanning device or sunlamp, made up of a large number of fluorescent tubes (2,3) which work with mercury vapour and low-pressure discharge. The fluorescent tubes (2,3) are alternated between pairs with a different radiation spectrum, their power varies between 40 and 200 W, and they emit up to 5% ultraviolet B compared to ultraviolet A. The wavelength of the ultraviolet A is between 315 and 400mm while that of the ultraviolet b is between 250 and 315 mm.

## Description

As expressed in the statement to this descriptive report, this invention consists of a progressive tanning system designed to be used for both aesthetic and health improvement purposes.

The benefits to living organisms of solar energy are well known, to the point that we cannot imagine life on earth without the sun. Our bodies need radiation from the sun in order to function properly. However, the latest information on the destruction of the ozone layer shows that direct exposure to the sun's rays can be very damaging. Therefore, people are increasingly choosing, for this or other reasons, the safety of artificial sunlight which, thanks to recent advances, poses fewer risks to the skin than natural light.

Sunlight is made up of rays of different wavelengths, which are divided into four types from short to long:
- Short-wave ultraviolet (UV.C) rays which have a germicidal effect and never reach the earth's surface.
- Ultraviolet B (UV.B) rays which are responsible for the production of melanin and for inducing erythema or reddening.
- Ultraviolet A (UV.A) rays, responsible for the darkening of the pigments.
- Infrared radiation (IR) which is responsible for heating up the skin on contact and for oxidizing the melanin which carries blood and, consequently, oxygen.

Artificial tanning techniques imitate the natural function of the sun's rays while trying to make the most of the advantages and lessening the disadvantages. Sunlamps, like the sun, emit ultraviolet radiation, but to a lesser degree. Both artificial and natural light irradiate type A ultraviolet (UV-A) radiation which is responsible for various photochemical and biological processes such as darkening the pigments in skin. The difference lies in the amount of ultraviolet B (UV-B) radiation, responsible for skin reddening, burns or erythema, which is much lower in sunlamps.

Natural sunlight and sunlamps share certain benefits. Both contribute to an improvement in the general state of health by preventing and treating problems such as psoriasis; concealing skin trouble; and palliating rheumatism, the decalcification of bones, muscular pain and, even, depression. Artificial sunlight is also beneficial because it stimulates the immune system and has certain positive effects on metabolism through modulating the photosynthesis of vitamin D3.
Furthermore, sunlamps provide a number of advantages over solar radiation, as detailed below:
- dosage (using a timer adjusted for one's phototype, the option of varying the amount of radiation with a personalised tanning program, avoiding dangerous levels of exposure);
- their spectral composition does not depend on the latitude, season or time of day;
- they emit less UV.B radiation which is responsible for skin reddening;
- they provide natural protection against future exposure to the sun;
- they can form part of a beauty treatment or be combined with the practice of some sport. Their relaxing, reconstructive and, finally, beauty effects make them an ideal complement to one's daily routine, and they can be used at any time of day or year and anywhere.

Since the first sunlamps were designed (from filterless mercury vapour lamps to the fluorescent lamps of the 70s and high-pressure lamps) the development of this type of device has focused on three aspects: treatment efficiency, minimization of exposure times to radiation and energy usage. Thus, today's high-density devices (highly packed with lots of tubes) are vastly improved in terms of their efficiency and reduced energy consumption. However, throughout this development, the amount of consideration placed on the safety of the radiation has not been the same, resulting in institutional measures having to be introduced in order to control the development.

Today, tanning devices are subject to two European directives: the Low-Voltage Directive 73/23/EEC and the Electromagnetic Compatibility Directive 89/336/EEC. These directives, whose aim is to regulate the manufacture of these devices in order to ensure that they are safe to use, affect matters such as safety distances, exposure times, stability, equipment classification, etc. In addition, government administrations regulate other technical aspects to control the effective irradiance, which cannot be below 0.3W/m², and wavelength, which cannot be less than 295 nm.

These new regulations represent significant advances in terms of safety but a clear setback in terms of the existing features of current tanning techniques.

In response to these regulations, and with the aim of achieving the same tanning features which have been effective until now, manufacturers have chosen to eliminate the UV.B (responsible for creating more melanin and inducing erythema or reddening) in the tubes. Direct or immediate pigmentation (caused by the UV.A) is greater but the resulting tans are much shorter lasting. As there is no stimulation of melanin production, only the body's existing pigments are tanned. Moreover, as many of these manufacturers maintain the same exposure times, the result is a lower-quality tan.

The new progressive tanning system is absolutely scrupulous in its compliance with prevailing legislation and, at the same time, noticeably improves the technique for tanning without UV.B, achieving a more healthy tan, with excellent direct pigmentation and optimal persistent pigmentation. As well as tanning and creating more melanin, it protects the skin from more aggressive exposure to natural sunlight.

To achieve this, the progressive tanning system consists of a vertical or horizontal structure made up of fluorescent tubes with two different types of α and β radiation spectrum, which operate with mercury vapour and low-pressure discharge, whose power oscillates between 40 and 200W, and which emit up to 5% ultraviolet B compared to ultraviolet A, where the wavelength of the UV.A is between 315 and 400 nm and the wavelength of the UV.B is between 295 and 315 nm. The progressive tanning system also has an on-off function and a system for programming the session time, cooling time and phototype.

This innovative tanning system alternates between one tube of up to 0.5% UV.B compared to UV.A and another which contains up to 2.6% UV.B compared to UV.A, which, working together, have a radiation level of less than 0.3 W/m², thus resulting in a progressive, healthy and long-lasting tan.

The characteristics of this tanning system ensure that energy consumption is reduced by 20%, which will represent a large saving for beauty and specialist centres in this sector.

To illustrate everything explained so far, a page of drawings is attached to this descriptive report, forming an integral part of it, where:

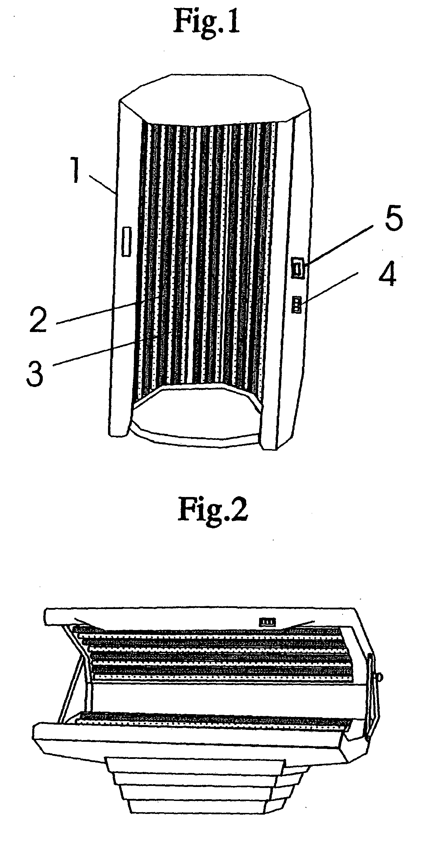

Figure 1 represents a progressive tanning system in a vertical machine.
Figure 2 represents a progressive tanning system in a horizontal machine.

The progressive tanning system consists of a vertical or horizontal structure (the one shown in the figure is a vertical one (1)) made up of a large number of two types of fluorescent tubes emitting α radiation (2) and β radiation (3). These mercury vapour, low-pressure discharge fluorescent tubes, whose power oscillates between 40 and 200 W, emit up to 5% UV.B compared to UV.A. The wavelength of the UV.A rays is between 315 and 400 nm and that of the UV.B rays is between 295 and 315 nm. The device has an on/off system (4), and a system for programming the exposure time, cooling time and phototype (5).

The materials used in the manufacture of the components that make up the artificial tanning system (sunlamp), and its shape, dimensions and accessories, are not part of the subject matter of this invention and may be replaced by others with the same technical specifications, as long as they do not essentially affect or depart from the scope defined in the claims outlined below.

## Claims

1. "PROGRESSIVE TANNING SYSTEM" of the type consisting of an artificial tanning device or sunlamp with a horizontal or vertical structure, comprised of a large number of fluorescent tubes which operate with mercury vapour and low-pressure discharge, **characterised by** the fact that it alternates pairs of fluorescent tubes with a different α and β radiation spectrum, whose power oscillates between 40 and 200 W, and which emit up to 5% ultraviolet B compared to ultraviolet A, where the wavelength of the ultraviolet A is between 315 and 400 nm while that of the ultraviolet B is between 250 and 315 nm.

2. "PROGRESSIVE TANNING SYSTEM", as per the claim above, **characterised by** the fact that each pair of tubes combines one tube of up to 0.5% ultraviolet B compared to ultraviolet A and another that contains up to 2.6% ultraviolet B compared to ultraviolet A, in such a way that, working together, the level of radiation is below 0.3 W/m².
